# EUROPEAN PATENT SPECIFICATION

(11) **EP 3 928 772 B1**
(45) Date of publication and mention of the grant of the patent: **19.06.2024**
(21) Application number: 20182425.7
(22) Date of filing: 26.06.2020
(51) Int. Cl.: A61K 9/51, A61K 9/16, A61K 31/00, A61K 31/167

(54) **NANOPARTICULATE COMPOSITION**
NANOPARTIKULÄRE ZUSAMMENSETZUNG
COMPOSITION NANOPARTICULAIRE

(43) Date of publication of application: 29.12.2021
(73) Proprietor: Algiax Pharmaceuticals GmbH, 40699 Erkrath (DE)
(72) Inventor: HASSE, Birgit, 42553 Velbert (DE); KOOPMANS, Guido, 6135 JC Sittard (NL); LIEBICH, Lena, 79539 Lörrach (DE); BÖGERSHAUSEN, Ansgar, 79102 Freiburg (DE); KNEISEL, Sandra, 79288 Gottenheim (DE); HAGEDORN, Martin, 79206 Breisach (DE); RISCHER, Matthias, 60388 Frankfurt (DE)
(74) Representative: Roth, Andy Stefan

(56) References cited:
- EP-A2- 2 632 451
- US-A1- 2010 297 252

## Description

### FIELD OF THE INVENTION

The present disclosure relates to a pharmaceutical composition comprising an active ingredient having an improved bioavailability and onset of action. Furthermore, the present invention relates to a pharmaceutical composition comprising said composition and the use of said composition for the preparation of a medicament Finally, the present invention relates to a method of making said composition and a system for the treatment of certain diseases using said composition.

### BACKGROUND OF THE INVENTION

### A. Background Regarding Nanoparticulate Compositions

US Pat. No. 5,145,684 describes nanoparticulate compositions comprising particles of a poorly soluble therapeutic or diagnostic agent. A non-crosslinked surface stabilizer is associated with the surface thereof.

Methods of making nanoparticulate compositions are described, for example, in US Pat. Nos. 5,518,187 and 5,862,999, both for "Method of Grinding Pharmaceutical Substances;" U.S. Pat. No. 5,718,388, for "Continuous Method of Grinding Pharmaceutical Substances;" and US. Pat. No. 5,510,118 for "Process of Preparing Therapeutic Compositions Containing Nanoparticles."

There are a number of nanoparticulate formulations listed under several patents for specific active pharmaceutical ingredients. WO 03/103633 describes nanoparticulate compositions comprising one or more sterols or stanols such as sitosterol or phytosterol. US 2008/02133374 A1 describes nanoparticulate compositions comprising sorafenib or a salt of sorafenib. US 2009/0238867 A1 describes nanoparticulate compositions comprising anidulafungin. U.S. 2008/0317843 A1 is directed to nanoparticulate compositions comprising modafinil. EP 1 658 053 describes novel nanoparticulate compositions of Sildenafil free base. WO 03/066021 A2 is directed to nanoparticulate compositions comprising lysozyme as a surface stabilizer. Another WO patent application is directed to nanoparticulate compositions comprising at least one poorly soluble MAP Kinase inhibitor and at least one surface stabilizer. US 2008/00220075 A1 describes nanoparticulate compositions comprising at least one poorly soluble angiogenesis inhibitor and at least one surface stabilizer. US 2003/0224058 A1 and US 2008/0241070 A1 are directed to fibrate compositions and their in-vivo behaviour in which the fibrate particles have an average particle size of less than about 2000 nm. US 2005/0095297 A1 discloses nanoparticulate formulations or suspensions comprising fibrate and Vitamin E TPGS. US 2019/0117674 A1 is directed to nanoparticulate compositions of Ganaxolone with a mean diameter between 50 and 500 nm. US 2018/0228810 A1 discloses nanoparticulate compositions comprising mexoxicam particles having a particle size of less than about 2000 nm. US 2018/0008601 A1 is directed to nanoparticulate forms of piperazine compounds. US 2015/0320682 A1 is directed to nanoparticulate compositions comprising megestrol. US 2013/0243830 A1 relates to nanoparticulate compositions containing corticosteroid compounds.

US 6,316,029 B1 is directed to rapidly disintegrating solid oral dosage forms of a poorly soluble active ingredient and at least one pharmaceutical acceptable water-soluble or water-dispersible excipient wherein the poorly soluble active ingredient particles have an average diameter of less than 2000 nm.

WO 03/024424 A1 and US 2012/0087984 A1 describe methods for stabilizing active ingredients, particularly pharmaceutical ingredients by forming active ingredients into a nanoparticulate composition comprising the active ingredient and at least one surface stabilizer.

WO 02/094215 A2 is directed to nanoparticulate compositions of poorly soluble drugs and at least one copolymer of vinyl pyrrolidone and vinyl acetate as a surface stabilizer adsorbed on the surface of the drug.

US 2002/0110597 A1 and US 6,375,986 B1 disclose solid nanoparticulate compositions comprising of a poorly soluble active agent, at least one polymeric surface stabilizer and dioctyl sodium sulfosuccinate (DOSS).

US 20020012675 is directed to controlled release of nanoparticulate compositions comprising a nanoparticulate agent and a rate-controlling polymer, which releases the agent after administration between 2 and 24 hours or longer.

EP 2632451 A2 discloses compositions comprising laflunimus or manitimus and excipients. The compositions may be in form of microspheres. The compositions are for treating neuropatic pain and inflammatory diseases.

### B. Background regarding (Z)-2-cyano-3-cyclopropyl-3-hydroxy-N-(3-methyl-4-trifluoromethyl)phenyl) prop-2-enamide (AP-325 - INN: Laflunimus)

(Z)-2-cyano-3-cyclopropyl-3-hydroxy-N-(3-methyl-4-trifluoromethyl)phenyl) prop-2-enamide (in the following also Laflunimus (INN) or AP-325 (working name)) and its derivatives belong to a class of compounds which are useful in the treatment of central nervous system (CNS)-trauma related disorders. Other compounds with such an activity are described in US 2016/022688 A1.
AP-325 (Laflunimus) has the following structure according to formula I: 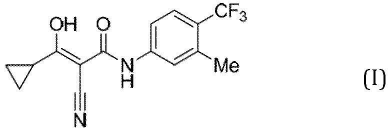 Similar useful compounds, which may also be understood as derivatives of AP-325, are:
(Z)-2-cyano-3-hydroxy-N-[4-(trifluoromethyl)phenyl]hept-2-en-6-ynamide (FK778 - INN: Manitimus) according to the following formula II 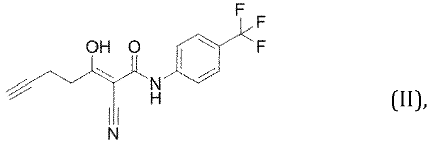 and 2-cyano-3-cyclopropyl-N-(4-fluorophenyl)-3-hydroxyacrylamide according to the following formula III:

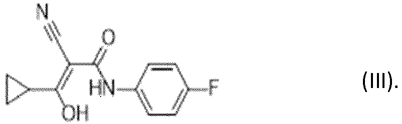

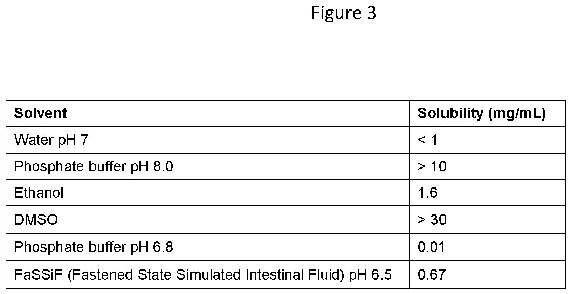

AP-325 is a pharmaceutical agent, which is currently in clinical development. Based on the solubility of the drug (see figure 3), AP-325 can be considered as practically insoluble at a physiological pH of 6.8 (phosphate buffer). For a better characterization of active ingredients with low solubility, a classification according to the BCS system (= Biopharmaceutics Classification System) was introduced (Guidance for Industry: Immediate release solid oral dosage forms and FDA, 1995). This BCS system distinguishes four categories based on the solubility and permeability of the active substance. At a pH value of below 7 AP-325 can be classified as a BCS class II drug substance. The low solubility of AP-325 negatively affects the absorption into the body after oral administration due to a limited absorption of the active agent in the small intestine (such as the jejunum or ileum), which is an essential absorption window for the uptake of a drug. Consequently, the therapeutic effect cannot be achieved.

### SUMMARY OF THE INVENTION

The present invention relates to a nanoparticulate composition comprising particles of at least one active ingredient selected from the group consisting of (Z)-2-cyano-3-cyclopropyl-3-hydroxy-N-(3-methyl-4-trifluoromethyl]phenyl] prop-2-enamide (Laflunimus, AP-325) in form of the free base, (Z)-2-cyano-3-hydroxy-*N*-[4-(trifluoromethyl)phenyl]hept-2-en-6-ynamide, 2-cyano-3-cyclopropyl-N-(4-fluorophenyl)-3-hydroxyacrylamide, and salts thereof, wherein the particles have an effective average particle size in the range from about 70 nm to about 220 nm. The composition of the invention comprises at least one surface stabilizer and/or at least one polymeric stabilizer.
Thus, the present invention essentially relates to the following three chemical compounds or active ingredients:
- (Z)-2-cyano-3-cyclopropyl-3-hydroxy-N-(3-methyl-4-(trifluoromethyl)phenyl) prop-2-enamide according to above formula I, also known as Laflunimus (INN) and preferably designated as AP-325 in the present patent specification; and/or
- (Z)-2-cyano-3-hydroxy-*N*-[4-(trifluoromethyl)phenyl]hept-2-en-6-ynamide, also known as Manitimus (INN) and preferably designated as FK778 in the present patent specification; and/or
- 2-cyano-3-cyclopropyl-N-(4-fluorophenyl)-3-hydroxyacrylamide
Furthermore, the present invention also relates to salts thereof.

The compositions of the invention show a surprisingly substantially improved bioavailability and on-set of action of the at least one active ingredient compared to a pharmaceutical dosage form comprising the active pharmaceutically ingredient having a particle size above 2 microns.

Another aspect of the invention is directed to pharmaceutical compositions comprising the above described composition in combination with at least one pharmaceutically acceptable excipient. The compositions also comprise at least one surface stabilizer and/or at least one polymeric stabilizer associated with the particles of the active ingredient.

This invention is also directed to a method of making the above described composition. Such a method may comprise contacting particles of the at least one active ingredient with at least one surface stabilizer and/or at least one polymeric stabilizer for a time and under conditions sufficient to provide a composition comprising particles of the active ingredient having an effective average particle size in the range from about 70 nm to about 220 nm. The one or more surface stabilizers and/or the one or more polymeric stabilizers can be contacted with the active ingredient either before, preferably during, or after particle size reduction.
This invention is also directed to a method of making a solid oral dosage form in which the nanoparticles from the nanosuspensions containing the at least one active ingredient as described for the above composition are bound on a suitable pharmaceutical excipient or carrier by using a fluid bed drying process, a spray drying process, an extrusion process or a granulation process.

This invention also relates to a pharmaceutical system for the treatment of certain diseases in a subject comprising administering to a subject of an effective amount of the above described composition.
This invention also relates to a pharmaceutical system containing particles of at least one active ingredient, e.g. AP-325, having an effective average particle size in the range from about 70 nm to about 220 nm which show a significant increase of the AUC (Area Under the Curve) of the active ingredient, e.g. AP-325, in the blood plasma and thereof a significant increase of the bioavailability compared to pharmaceutical systems containing particles of the active ingredient, e.g. AP-325, having an average particle size above 2 microns.

This invention is further directed to a pharmaceutical system containing particles of at least one active ingredient, e.g. AP-325, having an effective average particle size in the range from about 70 nm to about 220 nm which show a significant decrease of the Tₘₐₓ of the active ingredient, e.g. AP-325, in the blood plasma and thereof a significant faster on-set of action of the active ingredient, e.g. AP-325, compared to pharmaceutical systems containing particles of the active ingredient, e.g. AP-325, having an average particle size above 2 microns.

### BRIEF DESCRIPTION OF THE FIGURES

**Figure 1a****:** *In-vitro-release* dissolution profiles of a nanoparticulate composition (capsule, example 7) of AP-325 over a period of 24 months at 25°C/60% r.h.. The dissolution data confirm a very good stability and fulfil the relevant requirements of the Ph. Eur. for oral dosage forms.
**Figure 1b****:** Stability data of capsule batch G0625K102 stored in HDPE bottles over a period of 24 months at 25°C/60% r.h.. The stability data demonstrate that the AP-325 nanoparticulate composition is unaffected under the ICH storage conditions to any changes of the investigated parameters and can be regarded as very stable over the investigated storage period.

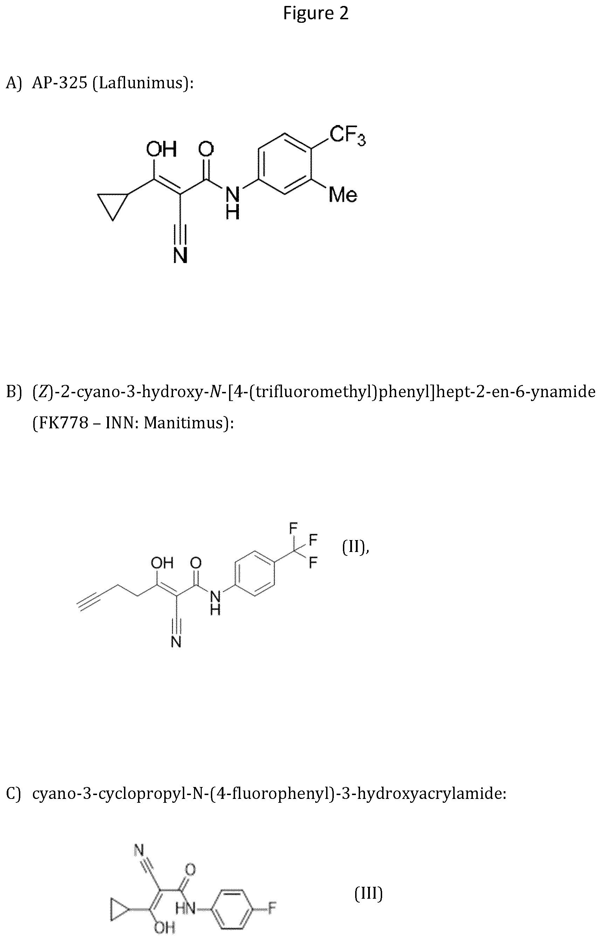

**Figure 2****:** Structural fomulas of AP-325 and its derivatives.
**Figure 3****:** Solubility of AP-325 in different solvents at ambient temperature. AP-325 can be considered as practically insoluble at a physiological pH of 6.8 (phosphate buffer).

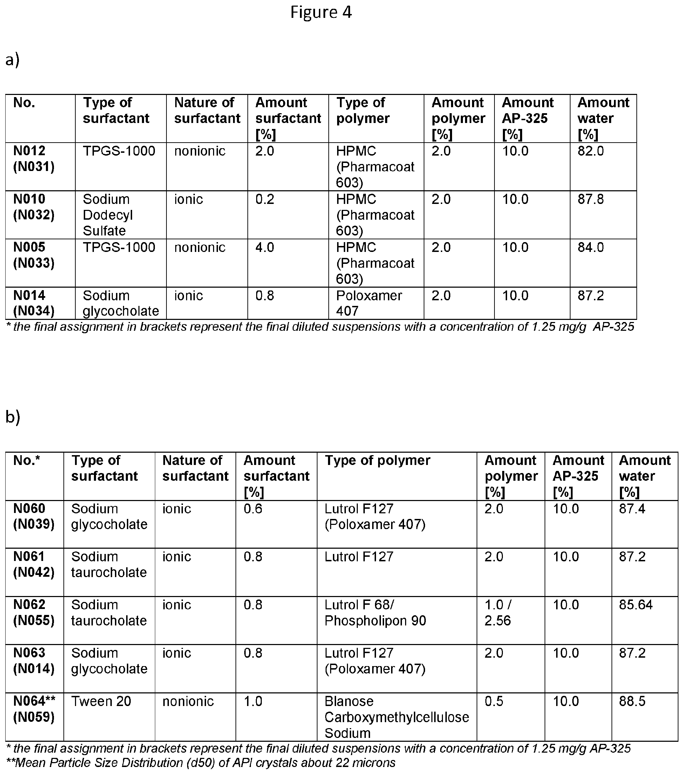

**Figure 4a****:** Nanoparticulate compositions of AP-325 applied in pk study in rats (study 1)
**Figure 4b****:** Nanoparticulate compositions of AP-325 applied in pk study in rats (study 2)
**Figure 5a****:** Tabulated results of pk study in rats (study 1). The nanoparticulate compositions of AP-325 exhibit increased bioavailability, at the same dose. Significant higher AUC and Cmax values for the preferable nanoparticulate composition (N014) of AP-325 can be reached compared to the microparticulate composition of AP-325 for the same dose.
**Figure 5b****:** Tabulated results of pk study in rats (study 2). Most nanoparticulate compositions have a significant faster Tmax (less than 2 h) than the composition containing AP-325 particles above 2 microns (Mikro N064). The nanoparticulate compositions of AP-325 out-performs their microparticulate counterparts on the pharmacokinetic parameters Tmax, Cmax and AUG.
**Figure 6****:** Tabulated results of pk study in dogs. The nanoparticulate composition showed a Tmax of 2 h whereas the composition containing AP-325 particles above 2 microns showed a Tmax of 2.5 h.
**Figure 7****:** Nanoparticulate compositions of AP-325 capsules applied in a pk study demonstrating a fast on-set of action and a dose-increasing good absorption in humans.
**Figure 8****:** XRPD data of dried AP-325 Nanosuspension, Bottom-line: Placebo, Lower middle-line: AP-325, Upper-line: Nanoparticulate formulation of AP-325, Upper middle-line: AP-325 suspended. All X-ray patterns remained unchanged after the milling process, proving that the crystallinity of the active ingredient AP-325 has not changed.
**Figure 9****:** Comparison of the particle size distribution (PSD) of G0625C101 at 25 °C/60% r.h. (granules / intermediate product of G0625K102). The PSD remained stable at storage conditions of 25°C/60% r.h. over a period of 24 months representing a very good stability of the nanoparticles of AP-325 in the dosage form.
**Figure 10****:** Comparison of the particle size distribution of G0625C101 at 40 °C/75% r.h. (granules / intermediate product of G0625K102). The PSD remained stable at storage conditions of 40°C/75% r.h. over a period of 6 months representing a very good stability of the nanoparticles of AP-325 in the dosage form.

### DETAILED DESCRIPTION OF THE INVENTION

### A. Additional preferred characteristics of the AP-325 nanoparticulate compositions of the invention

### 1. Fast On-set of Activity

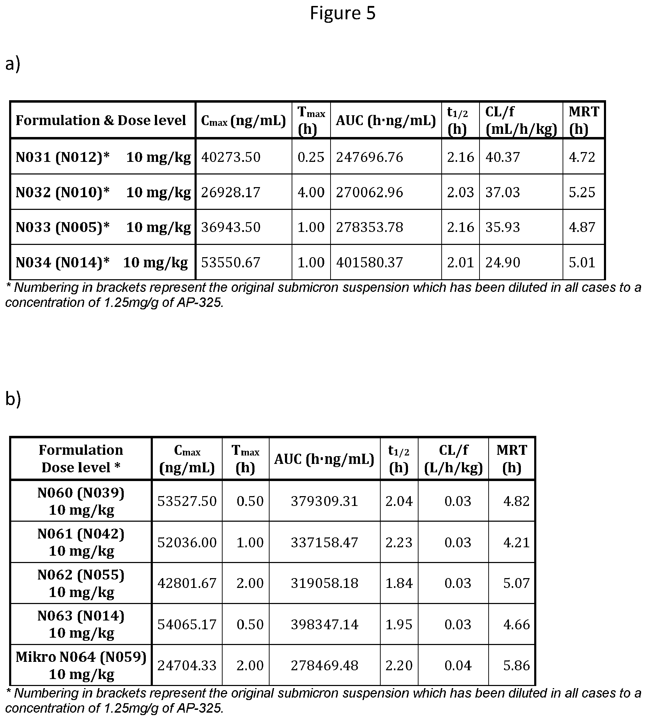

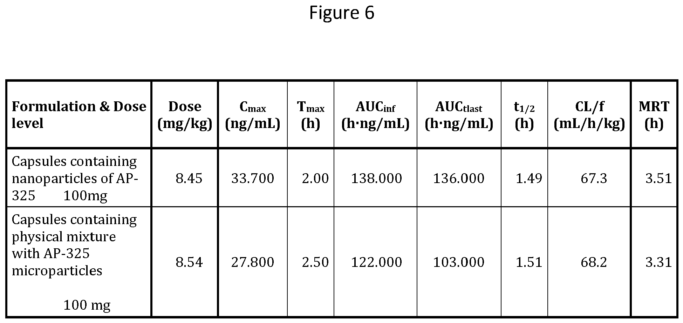

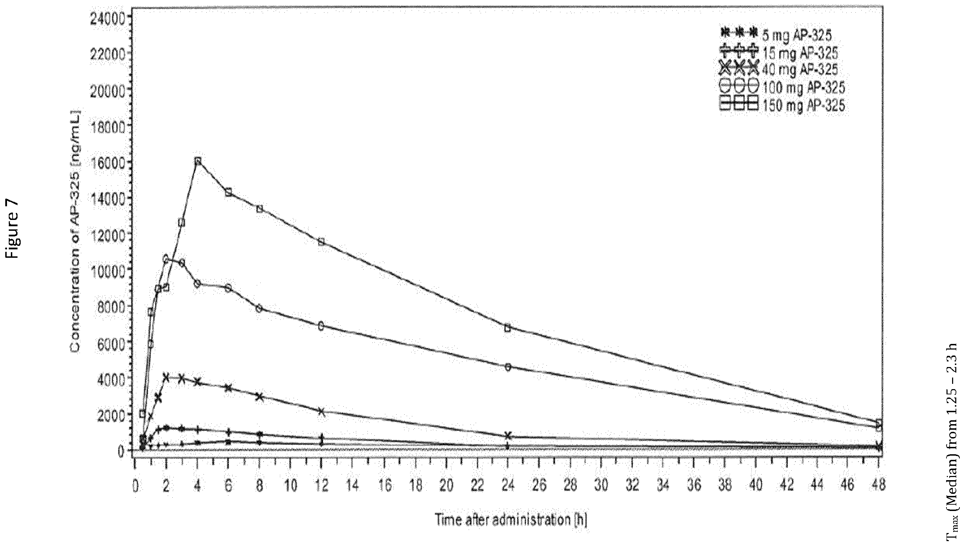

Nanoparticulate compositions of AP-325 show a fast on-set of action, especially compared to compositions of AP-325 which contain particles in the microns range above 2 microns. As demonstrated in figure 5b three of the tested nanoparticulate compositions in rats have a significant faster Tₘₐₓ (less than 2 h) than the composition containing AP-325 particles above 2 microns and only one nanoparticulate composition had the same Tₘₐₓ in this study (about 2 h). This observation was confirmed in the dog study presented in figures 6 and 7 in which the nanoparticulate composition showed a Tₘₐₓ of 2 h whereas the composition containing AP-325 particles above 2 microns showed a Tₘₐₓ of 2.5 h. Finally, the fast on-set of action has been also demonstrated for the nanoparticulate composition of AP-325 in a pk study in humans (see figure 7) in which a T_{max (median)} between 1.25 - 2.3 h was seen.

The demonstrated fast on-set of action is important in the treatment of the mentioned neuropathic pain and central nervous system trauma related disorder to represent a high level of compliance towards the patients.

### 2. Increased Bioavailability

The nanoparticulate compositions of AP-325 of the invention preferably exhibit increased bioavailability, at the same dose, and require smaller doses as compared to prior non-nanoparticulate AP-325 compositions. This was demonstrated in figure 5a) and 5b) for the nanoparticulate composition of AP-325 (N014/N063) to be superior over the non-nanoparticulate composition of AP-325 (N064/N059) with significant higher bioavailability of 147% in the nanoparticulate composition compared to 82% in the non-nanoparticulate composition of AP-325 and significant higher AUC and Cₘₐₓ values for the preferable nanoparticulate composition (N014) of AP-325 compared to the non-nanoparticulate composition of AP-325 for the same dose.

### 3. Pharmacokinetic Profiles of the AP-325 nanoparticulate compositions of the invention

The present invention provides nanoparticulate AP-325 compositions having a desirable pharmacokinetic profile when administered to mammalian subjects. The desirable pharmacokinetic profile can include one or more of the following characteristics: (1) the Tₘₐₓ of an administered dose of a nanoparticulate AP-325 composition can be less than that of a non-nanoparticulate AP-325 composition, (2) the Cₘₐₓ of a nanoparticulate AP-325 composition can be greater than the Cₘₐₓ of a non-nanoparticulate AP-325 composition, (3) the AUC of a nanoparticulate AP-325 composition can be greater than the AUC of a non-nanoparticulate AP-325 composition. This has been proven in comparative studies reported in figures 5a), 5b) and 7.

In a pk study with healthy volunteers with doses of 5 to 150 mg per patient a T_{max (median)} from 1.25 h (5 mg) to 2.3 h (150 mg), a Cₘₐₓ₍ₘₑₐₙ₎ from 475 ng/ml (5 mg) to 17961 ng/ml (150 mg) and an AUC of 10578 h·ng/mL (5 mg) to 381961 h·ng/mL (5 mg) was seen as shown in figure 7, demonstrating the fast on-set of action and a dose-increasing good absorption profile in human for this nanoparticulate composition of AP-325.

### B. Compositions

### 1. AP-325 Derivatives

AP-325 and further chemical compounds, which may be understood as AP-325 derivatives, are described in **[010]** and **[011].** Derivative (*Z*)-2-cyano-3-hydroxy-*N*-[4-(trifluoromethyl)phenyl]but-2-enamide/ Teriflunomide with its shown chemical structure is excluded from this patent:

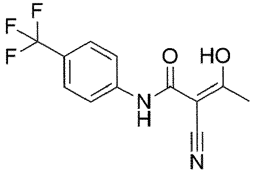

### 2. Surface and polymeric stabilizers

The choice of one or more surface and polymeric stabilizers for nanoparticulate compositions of AP-325 is non-trivial and required extensive experimentation to realize a desirable formulation. Combinations of more than one surface and/or polymeric stabilizer can be used in the invention. Useful surface stabilizers which can be employed in the invention include, but are not limited to, known organic and inorganic pharmaceutical excipients. Such excipients include various polymers, low molecular weight oligomers, natural products, and surfactants. Surface stabilizers include nonionic, cationic, ionic, and zwitterionic compounds whereas polymeric stabilizers include well-known polymers widely used in pharmaceutical compositions like cellulose derivatives, polysaccharides, polyethylene derivatives, phospholipids, alginates and the like.

Representative examples of surface and polymeric stabilizers include, but are not limited to, hydroxypropyl methylcellulose, hydroxypropylcellulose, sodium lauryl sulfate, gelatin, casein, lecithin (phosphatides), bile salts like taurocholate and derived salts like sodium glyocholate, dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, the commercially available Tweens such as e.g., Tween 20^{®} and Tween 80^{®}, polyethylene glycols, polyoxyethylene stearates, colloidal silicon dioxide, phosphates, carboxymethylcellulose calcium, carboxymethylcellulose sodium, methylcellulose, hydroxyethylcellulose, hydroxypropylmethylcellulose phthalate, noncrystalline cellulose, magnesium aluminium silicate, triethanolamine, polyvinyl alcohol (PVA), poloxamers (e.g., Pluronics F68^{®} and F127^{®}, which are block copolymers of ethylene oxide and propylene oxide); poloxamines (e.g., Tetronic 908^{®}, also known as Poloxamine 908^{®},which is a tetrafunctional block copolymer derived from sequential addition of propylene oxide and ethylene oxide to ethylenediamine, Tritons X-200^{®} which is an alkyl aryl polyether sulfonate; Crodestas F-110^{®}, which is a mixture of sucrose stearate and sucrose distearate, n-alkyl-beta-D-glucopyranosides; PEG-phospholipid, PEG-cholesterol, PEG-cholesterol derivative, PEG-vitamin A, PEG-vitamin E e.g. TPGS-1000^{®}, lysozyme, and the like.

### 3. Other Pharmaceutical Excipients

Pharmaceutical AP-325 nanoparticulate compositions according to the invention may also comprise one or more binding agents, filling agents, lubricating agents, suspending agents, sweeteners, flavoring agents, buffers, wetting agents, disintegrants, effervescent agents, and other excipients. Such excipients are known in the art

Suitable examples for starter pellets for fluid bed coating or granulation processes are lactose monohydrate, microcrystalline cellulose or isomalt

Examples of filling agents and diluents are mannitol, lactose monohydrate, lactose anhydrous, and various starches, dibasic calcium phosphate, saccharides and/or mixtures of any of the foregoing; examples of binding agents are various celluloses and cross-linked polyvinylpyrrolidone, microcrystalline cellulose, such as Avicel^{®} PH101 and Avicel^{®} PH102, and silicified microcrystalline cellulose (ProSolv SMCC^{™}).

Suitable lubricants, including agents that act on the flowability of a powder to be compressed, are colloidal silicon dioxide, such as Aerosil^{®} 200, talc, stearic acid, magnesium stearate, calcium stearate, and silica gel.

Suitable disintegrants include lightly crosslinked polyvinyl pyrrolidone, corn starch, potato starch, maize starch and modified starches, croscarmellose sodium, cross-povidone, sodium starch glycolate, and mixtures thereof.

Examples of effervescent agents are effervescent agents such as an organic acid and a carbonate or bicarbonate. Suitable organic acids include, for example, citric, tartaric, malic, fumaric, adipic, succinic, and alginic acids and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent agent may be present.

Examples of sweeteners are any natural or artificial sweetener, such as sucrose, xylitol, sodium saccharin, cyclamate, aspartame, and acesulfame

### . 4. Nanoparticulate AP-325 Active particle size distribution

The compositions of the invention include particles of one or several of the active ingredients, e.g. AP-325, which have an effective average particle size in the range from about 70 nm to about 220 nm, as measured by light-scattering methods. The effective average particle size is in the range from about 70 nm to about 220 nm, more preferably in the range from about 90 nm to about 210 nm, even more preferably in the range from about 100 nm to about 200 nm.

By "an effective average particle size of less than about 2000 nm" it is meant that at least 50% of said active agent particles have a particle size, by weight (volume based), of less than the effective average particle size when measured by the above-noted techniques, e.g., 50% of the particles have a size, by weight, of less than about 2 microns (or less than about 1900 nm, less than about 1800 nm, etc.).
In an advantageous embodiment, the particles are measured with a state-of-the-art anylytical technique like a static or dynamic laser diffraction method (e.g. Malvern Sizer or Zeta Sizer). For example, the use of laser light diffraction to measure particle size is a widely known technique. Laser diffraction is a particle sizing method which uses the average relative angular intensity of scattered light. Instruments that use laser light diffraction to measure particle size have been available for many years from a number of different manufacturers. All laser diffraction instruments use the same basic method to measure particle size. All laser diffraction instruments require a beam of monochromatic light with a very uniform wave front. This beam of laser light is directed at the sample particles to be measured. When the light hits the particles, the light is diffracted or scattered from the particles. Detectors are used to measure the relative average intensity of the light scattered at various angles from the sample material. Once the relative intensity of light scattered at several different angles from the particles is known, the particle size and size distribution can be calculated.

### 5. Concentration of nanoparticulate AP-325 and active and surface and/or polymeric stabilizer

The relative amounts of nanoparticulate AP-325 and one or more surface and/or polymeric stabilizers can vary widely. The optimal amount of the individual components can depend, for example, upon the hydrophilic lipophilic balance (HLB), melting point, pH dependent solubility, pKa values.

The concentration of AP-325 can vary from about 99.5% to about 0.001%, from about 95% to about 0.1%, or from about 90% to about 0.5%, by weight, based on the total combined dry weight of AP-325 and at least one surface and/or polymeric stabilizer, not including other excipients.

The concentration of at least one surface and/or polymeric stabilizer can vary from about 0.5% to about 99.9%, from about 5.0% to about 99.9%, or from about 10% to about 99.5%, by weight, based on the total combined dry weight of AP-325 and at least one surface and/or polymeric stabilizer, not including other excipients.

### C. Methods of making nanoparticulate AP-325 compositions

### 1. Milling to obtain nanoparticulate AP-325 compositions

Milling of AP-325 to obtain a nanoparticulate composition comprises dispersing particles of AP-325 in a liquid dispersion media in which AP-325 is poorly soluble, followed by applying mechanical means in the presence of hard grinding media to reduce the particle size of AP-325 to the desired effective average particle size. The dispersion media can be, for example, water, glycerine, polyethylene glycol (PEG), or glycol. Water is the preferred dispersion media.

The AP-325 particles are preferably reduced in size in the presence of at least one surface and/or polymeric stabilizer. Other compounds, such as a diluent, can be added to the AP-325 surface and polymeric stabilizer composition during the particle size reduction process. Dispersions can be manufactured continuously or in a batch mode.

### D. Methods of using the nanoparticulate AP-325 compositions of the invention

### 1. Treatment Applications

The AP-325 compositions of the invention are useful in treating and/or preventing, among other diseases and conditions, neuropathic pain and central nervous system trauma related disorders in humans.

The following examples are given to illustrate the present invention. It should be understood, however, that the invention is not limited to the specific conditions or details described in these examples.

### E. Examples

### 1. General milling conditions

The purpose of these examples was to show the application of the invented milling process for AP-325 and its derivatives in the laboratory scale to obtain fast results with limited amounts of active ingredient (screening phase) and in the pilot scale to demonstrate the suitability of the invented process for the manufacture of clinical trials samples.

**Lab scale trials:** The indicated amount of water (e.g. purified water) was weighed into a small grinding vessel made of zircon oxide (approx. 45 ml volume). Afterwards the given amounts of surfactant and stabilising polymer were added under stirring at ambient temperature until the components fully dissolved. Afterwards the described amount of AP-325 or its derivatives was slowly added under stirring to give an almost homogenous suspension. The magnetic bar was removed and an appropriate amount of yttria-stabilised zirconium milling beads in a suitable diameter (0.1 - 1.0 mm) was added to the suspension. The milling vessel was tightly closed and fixed in the laboratory nanomill chamber (e.g. Pulverisette 7 from Fritsch). Milling was started at an appropriate milling speed (e.g. 600 - 800 rpm) over an appropriate time interval (2-10 h) in a reverse mode function. After milling, the milling chamber was opened with care and the nanosuspension separated from the milling beads. The isolated nanosuspension was measured on particle size distribution (PSD) by a static laser diffraction (e.g. Malvern Mastersizer). Alternatively, the trials have been performed using a dual centrifugation system (e.g. Zentrimix 380) with 1 ml sample volume, with the same diameter range of milling beads and with a milling speed of about 1500 -2500 rpm over a period between 1 - 4hours.

**Pilot scale trials:** Initially the given amount of surfactant (e.g. sodium glycocholate) was dissolved in water (e.g. purified water) under stirring at ambient temperature. Afterwards the polymeric stabilizer (e.g. poloxamer 407) was added and fully dissolved in this solution. To get a homogeneous suspension, the AP-325 was incorporated step by step into the surfactant/polymer solution until all agglomerates of AP-325 were destroyed and a homogenous micro-suspension was obtained. Afterwards the micro-suspension was filled into the milling chamber of a suitable bead mill (e.g. Netzsch DeltaVita 300) and an appropriate amount of yttriastabilised zirconium milling beads (diameter from 0.1 - 1.0 mm) was added and the wet bead milling process was performed until the particle size distribution had reached the final requested nanometer range (several hours depending on the scale). A typical scale consisted of about 190 g of AP-325, about 1650g of water, about 38 g polymeric stabilizer (e.g. Kolliphor P 407) and about 15 g of surface stabilizer (e.g. sodium glycocholate).

### 2. Examples for nanoparticulate compositions (nanosuspensions) of AP-32 5 and its derivatives

Nanosuspensions of AP-325 were prepared in accordance to **[043]:**

**Table 1**

| **No.** | **Type of surfactant** | **Nature of surfactant** | **Amount surfactant [%]** | **Type of polymer** | **Amount polymer [%]** | **Amount AP-325 [%]** | **Amount water [%]** | **Total [%]** |
|---|---|---|---|---|---|---|---|---|
| **N005** | TPGS-1000 | nonionic | 4.0 | HPMC (Pharmacoat 603) | 2.0 | 10 | 84.00 | 100 |
| **N012** | TPGS-1000 | nonionic | 2.0 | HPMC (Pharmacoat 603) | 2.0 | 10 | 86.00 | 100 |
| **N014** | Sodium glycocholate | ionic | 0.8 | Poloxamer 407 (Lutrol F127) | 2.0 | 10 | 87.20 | 100 |
| **N039** | Sodium glycocholate | ionic | 0.6 | Poloxamer 407 (Lutrol F127) | 2.0 | 10 | 87.40 | 100 |
| **N040** | Sodium glycocholate | ionic | 0.6 | Poloxamer 188 (Lutrol F68) | 4.0 | 10 | 85.40 | 100 |
| **N041** | Sodium glycocholate | ionic | 0.6 | Poloxamer 407 (Lutrol F127) | 4.0 | 10 | 85.40 | 100 |
| **N042** | Sodium taurocholate | ionic | 0.8 | Poloxamer 407 (Lutrol F127) | 2.0 | 10 | 87.20 | 100 |
| **N043** | Sodium taurocholate | ionic | 0.8 | Poloxamer 188 (Lutrol F68) | 2.0 | 10 | 87.20 | 100 |
| **N045** | Sodium taurocholate | ionic | 0.8 | Poloxamer 188 (Lutrol F68) | 4.0 | 10 | 85.20 | 100 |
| **N055** | Sodium taurocholate | ionic | 0.8 | Poloxamer 188/ Phospholipon 90 | 1.0 / 2.56 | 10 | 85.64 | 100 |
| **N057** | TPGS-1000 | nonionic | 2.0 | Poloxamer 188 (Lutrol F68) | 4.0 | 10 | 84.00 | 100 |

Nanosuspensions of (Z)-2-cyano-3-hydroxy-*N*-[4-(trifluoromethyl)phenyl]hept-2-en-6-ynamide / FK778 / Manitimus were prepared in accordance to **[043]:**

**Table 2**

| **No.** | **Type of surfactant** | **Nature of surfactant** | **Amount surfactant [%]** | **Type of polymer** | **Amount polymer [%]** | **Amount AP-325 [%]** | **Amount water [%]** | **Total [%]** |
|---|---|---|---|---|---|---|---|---|
| **L001** | Sodium glycocholate | ionic | 0.8 | Poloxamer 407 (Lutrol F127) | 2.0 | 10 | 87.20 | 100 |
| **L002** | TPGS-1000 | nonionic | 4.0 | HPMC (Pharmacoat 603) | 2.0 | 10 | 84.00 | 100 |

Nanosuspension of 2-cyano-3-cyclopropyl-N-(4-fluorophenyl)-3-hydroxyacrylamide were prepared in accordance to **[043]:**

**Table 3**

| **No.** | **Type of surfactant** | **Nature of surfactant** | **Amount surfactant [%]** | **Type of polymer** | **Amount polymer [%]** | **Amount AP-325 [%]** | **Amount water [%]** | **Total [%]** |
|---|---|---|---|---|---|---|---|---|
| **L003** | Sodium glycocholate | ionic | 0.8 | Poloxamer 407 (Lutrol F127) | 2.0 | 10 | 87.20 | 100 |
| **L004** | TPGS-1000 | nonionic | 4.0 | HPMC (Pharmacoat 603) | 2.0 | 10 | 84.00 | 100 |

Nanosuspensions of AP-325 were prepared in accordance to **[044]:**

**Table 4**

| **No.** | **Type of surfactant** | **Nature of surfactant** | **Amount surfactant [%]** | **Type of polymer** | **Amount polymer [%]** | **Amount AP-325 [%]** | **Amount water [%]** |
|---|---|---|---|---|---|---|---|
| **N058** | Sodium glycocholate | ionic | 0.8 | Poloxamer 407 (Lutrol F127) | 2.0 | 10 | 87.20 |
| **N071** | Sodium glycocholate | ionic | 0.8 | Poloxamer 407 (Lutrol F127) | 2.0 | 10 | 87.20 |
| **N072** | Sodium glycocholate | ionic | 0.8 | Poloxamer 407 (Lutrol F127) | 2.0 | 10 | 87.20 |
| **N073** | Sodium glycocholate | ionic | 0.8 | Poloxamer 407 (Lutrol F127) | 2.0 | 10 | 87.20 |
| **N076** | Sodium glycocholate | ionic | 0.8 | Poloxamer 407 (Lutrol F127) | 2.0 | 10 | 87.20 |
| **N077** | Sodium glycocholate | ionic | 0.8 | Poloxamer 407 (Lutrol F127) | 2.0 | 10 | 87.20 |

**3. Particle size distribution of nanosuspensions containing AP-325 and derivatives, stability and prove of crystallinity**; nanoparticulates not falling in the range from about 70 nm to about 220 nm are not part of the invention and can be seen as reference examples.

Particle size distribution was measured by laser light scattering (e.g. Malvern Mastersizer) for the nanosuspensions of AP-325 obtained under **[045]:**

**Table 5**

| **No.** | **D10 [µm]** | **D50 [µm]** | **D 90 [µm]** |
|---|---|---|---|
| **N005** | 0.07 | 0.12 | 0.20 |
| **N012** | 0.07 | 0.12 | 0.21 |
| **N014** | 0.07 | 0.14 | 0.91 |
| **N039** | 0.07 | 0.14 | 1.11 |
| **N040** | 0.07 | 0.13 | 0.54 |
| **N041** | 0.07 | 0.14 | 0.81 |
| **N042** | 0.08 | 0.13 | 0.30 |
| **N043** | 0.08 | 0.13 | 0.23 |
| **N045** | 0.08 | 0.14 | 0.28 |
| **N055** | 0.07 | 0.14 | 1.25 |
| **N057** | 0.07 | 0.14 | 1.05 |

Particle size distribution was measured by laser light scattering (e.g. Malvern Mastersizer) for the nanosuspensions of (Z)-2-cyano-3-hydroxy-*N*-[4-(trifluoromethyl)phenyl]hept-2-en-6-ynamide / FK778 / Manitimus obtained under **[046]:**

**Table 6**

| **No.** | **D10 [nm]** | **D50 [nm]** | **D90 [nm]** |
|---|---|---|---|
| **L001** | 81 | 271 | 1911 |
| **L002** | 71 | 133 | 317 |

Particle size distribution was measured by laser light scattering (e.g. Malvern Mastersizer) for the nanosuspensions of 2-cyano-3-cyclopropyl-N-(4-fluorophenyl)-3-hydroxyacrylamide obtained under **[047]:**

**Table 7**

| **No.** | **D10 [nm]** | **D50 [nm]** | **D90 [nm]** |
|---|---|---|---|
| **L003** | 27 | 117 | 532 |
| **L004** | 24 | 95.2 | 1210 |

Particle size distribution was measured by laser light scattering (e.g. Malvern Mastersizer) for the nanosuspensions of AP-325 obtained under **[048]**:

**Table 8**

| **No**. | **D10 [µm]** | **D50 [µm]** | **D90 [µm]** |
|---|---|---|---|
| **N058** | 0.07 | 0.13 | 0.29 |
| **N071** | 0.07 | 0.14 | 0.34 |
| **N072** | 0.07 | 0.14 | 0.44 |
| **N073** | 0.07 | 0.15 | 0.43 |
| **N076** | 0.07 | 0.15 | 0.48 |
| **N077** | 0.07 | 0.15 | 0.47 |

Particle size distribution (PSD) was measured by laser light scattering (e.g. Malvern Mastersizer) for the nanosuspensions of AP-325 obtained under **[048]** and have been measured again after certain time intervals and storage conditions (temperature/humidity). The results confirmed that the PSD was unchanged over the investigated time intervals and conditions confirming the good stability of the nanoparticulate composition of AP-325.

### Condition: 2-8°C (refrigerator) / Batch N077

**Table 9**

| **No.** | **D10 [µm]** | **D50 [µm]** | **D90 [µm]** |
|---|---|---|---|
| 1 week | 0.07 | 0.15 | 0.52 |
| 2 weeks | 0.07 | 0.15 | 0.49 |
| 4 weeks | 0.07 | 0.15 | 0.49 |
| 12 weeks | 0.07 | 0.15 | 0.50 |

### Condition: 25°C/60% r.h. (climate chamber) /Batch N077

**Table 10**

| **No.** | **D10 [µm]** | **D50 [µm]** | **D90 [µm]** |
|---|---|---|---|
| 1 week | 0.07 | 0.17 | 0.95 |
| 2 weeks | 0.07 | 0.17 | 0.87 |
| 4 weeks | 0.07 | 0.17 | 0.91 |
| 12 weeks | 0.07 | 0.16 | 0.88 |

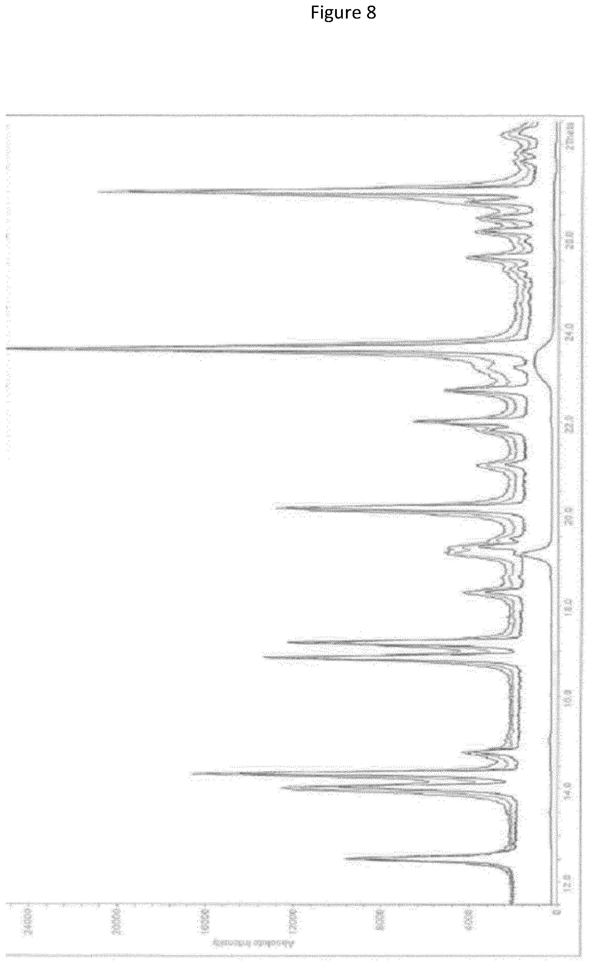

It is important that during the milling process no change of the crystallinity of the active ingredient occurs (e.g. change of a polymeric form). To prove that the crystallinity of the active ingredient remained unchanged, a part of suspension of N073 had been dried and analysed by powder X-ray diffraction (PXRD; e.g. STOE Stadi P Transmission mode, Cu-K alpha irradiation) against the pure active ingredient, the placebo mixture and the active ingredient suspended and dried. As it can be seen in figure 8 all X-ray patterns remained unchanged after the milling process, proving that the crystallinity of the active ingredient AP-325 has not changed.

It is further important that the obtained nanoparticulate compositions of AP-325 are stable in the acidic environment as otherwise the "nanoeffect" would simply disappear if the nanoparticles would be converted into microcrystals above 2000 nm under such conditions. In order to prove the stability in acidic environment the nanosuspensions of AP-325 have been treated with 0.06 M hydrochloric acid (pH = 1.2) over a certain period (e.g. 1-2 hours) at 37°C. The data shown in table 11 prove the good stability of the invented nanosuspensions of AP-325 under acidic conditions:

**Table 11**

| **Batch No.** | **D10 [µm]** | **D50 [µm]** | **D90 [µm]** |
|---|---|---|---|
| **N014** | 0.07 | 0.14 | 0.79 |
| **N022 (=N012)** | 0.07 | 0.12 | 0.21 |
| **N024 (= N005)** | 0.07 | 0.12 | 0.20 |

Degradation of the active ingredient may occur during the milling process. In order to prove that no significant degradation of AP-325 was obtained the nanosuspension has been investigated after milling by a specific, validated HPLC method (e.g. using a HPLC Agilent 1290 instrument). The results in table 12 prove that no degradation could be detected after the milling process, all impurities remained at a very low level:

**Table 12**

| **Batch** | **N005** | **N012** | **N014** |
|---|---|---|---|
| **Colour of content** | white | white | white |
| **Purity** | | | |
| **COPR1 (RRT 0.50)** | - | - | - |
| **COPR2 (RRT 0.57)** | - | 0.05% | 0.05% |
| **unknown (RRT 0.67)** | - | 0.05% | - |
| **unknown (RRT 0.86)** | - | - | - |
| **Sum of impurities** | - | 0.10% | 0.05% |

Investigations had also been made to detect residual traces of zirconium and yttrium from the milling beads collision during the milling process confirming that only very low traces in the low ppm range for both elements were obtained in the nanosuspensions of AP-325 indicating the suitability of the invented milling process for its intended use.

### 4. Examples for granulate compositions containing nanoparticles of AP-325

The nanosuspension of AP-325 obtained under [**044**] were converted into a dosage form in which the nanoparticles are bound on a suitable carrier in order to prevent the so called Ostwald ripening effect of the nanoparticles in the nanosuspension itself. The compositions shown are only one possibility of converting the nanoparticles into a powder formulation and other techniques like spray drying, extrusion, direct granulation may be used as well.

**Fluid Bed Layering:** To the nanosuspensions obtained under [44] a suitable polymeric stabilizer is added under stirring at ambient temperature until fully dissolved. Further, the completed layering suspension containing AP-325 was layered on s suitable carrier in a fluid bed coater (e.g. Unilab from Bosch with bottom spray nozzles). For the layering process standard nozzles and filters have been used, the inlet temperature was set to max. 60°C to get a product temperature of about 40°C. The spraying pressure was set less than 1 bar. A typical scale consisted of about 280g Nanosuspension obtained under **[044]**, about 9 g of additional polymeric stabilizer (e.g. Hypromellose 6mPas) and about 1220g of carrier (e.g. Isomalt galenIQ 960). The process was completed within several hours, depending on the scale.

**Final blending:** The final dried pellets from [58] were blended with suitable lubricants for several minutes in a suitable blender at ambient temperature to avoid sticking during the capsule filling process. A typical scale consisted of about 800g pellets containing nanoparticles of AP-325 and about 18 g of a lubricant (e.g. Talc).

**Encapsulation:** The mixture is filled into hard gelatine capsules (e.g. size 0) by pellet dosing unit of the capsule filling machine (e.g. Bonapace capsule filling machine). Table 13 gives an overview of different batches which had been produced in accordance to **[058], [059], [060]** and **[061].**

**Table 13**

| **Batch** | **Granule batch** | **Amount Granules [mg]** | **Amount Talc** | **Filling weight [mg]** | **Dose [mg]** |
|---|---|---|---|---|---|
| K001 | C012 | 214.8 | 5.7 | 220.5 | 5 |
| K002 | C012 | 107.4 | 2.5 | 109.9 | 2.5 |
| K003 | C010 | 237.8 | 5.0 | 242.8 | 25 |
| K004 | C011 | 214.6 | 5.0 | 219.6 | 25 |
| K006 | C014 | 219.4 | 5.0 | 224.4 | 5 |
| K007 | C014 | 109.7 | 2.5 | 112.2 | 2.5 |

**Composition of a capsule:** Typical compositions of capsules formulations in accordance to this invention are represented in tables 14 and 15:

**Table 14**

| One capsule contains (based on a theoretical assay of 100% granules) for the *25 mg strength:* | |
|---|---|
| **Ingredient** | **Quantity** |
| AP-325 | 25.00 mg |
| Poloxamer 407 | 5.00 mg |
| Sodium glycocholate | 2.00 mg |
| Hydroxypropylmethylcellulose E6 (6 mPas) | 8.00 mg |
| Isomalt | 218.00 mg |
| Talc | 5.00 mg |

**Table 15**

| One capsule contains (based on a theoretical assay of 100% granules) for the *5 mg strength:* | |
|---|---|
| **Ingredient** | **Quantity** |
| AP-325 | 5 mg |
| Poloxamer 407 | 1.00 mg |
| Sodium glycocholate | 0.40 mg |
| Hydroxypropylmethylcellulose E6 (6mPas) | 1.60 mg |
| Isomalt | 218.00 mg |
| Talc | 5.00 mg |

### 5. Particle size and dissolution profiles of nanoparticulate AP-325 composition in hard gelatine capsules

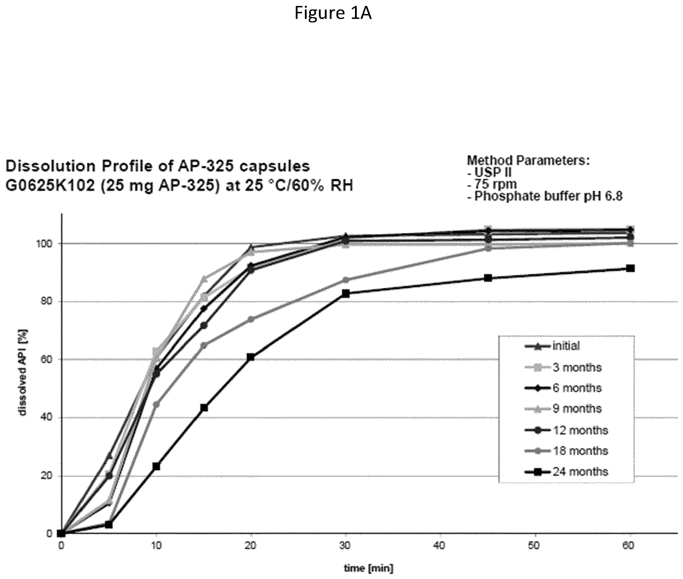

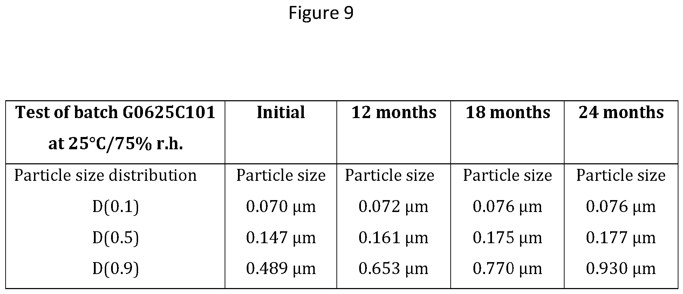

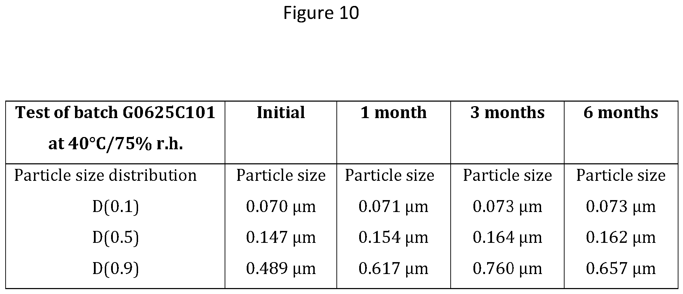

Important parameters for nanoparticulate compositions during the stability testing under different conditions are that the particle size of the nanoparticles of AP-325 and that the derived dissolution profiles in physiological media remained unchanged. Figure 9 and figure 10 show the particle size behaviour during stability testing at different conditions. As a water insoluble lubricant (talc) had been used in the formulations the particle size measurements had been done for the granules of the corresponding batch as the lubricant is interfering with the PSD measurements with the laser light scattering techniques. The results demonstrate that the PSD remained unchanged at storage conditions of 25°C/60% r.h. and 40°C/75% r.h. over a period of 24 respective 6 months representing a very good stability of the nanoparticles of AP-325 in the dosage form. This good stability is also confirmed by the dissolution data presented in figure 1a over a period of 24 months at 25°C/60% r.h at 37°C in physiological phosphate buffer. The small drop of the dissolution profile at 18 and 24 months is obviously related to a small change (e.g. sticking) of the granules within the capsules, but the overall release is still well above 80% after 60 minutes and fulfils the relevant requirements of the Ph. Eur. for oral dosage forms.

### 6. Stability data of nanoparticulate AP-325 composition in hard gelatine capsules

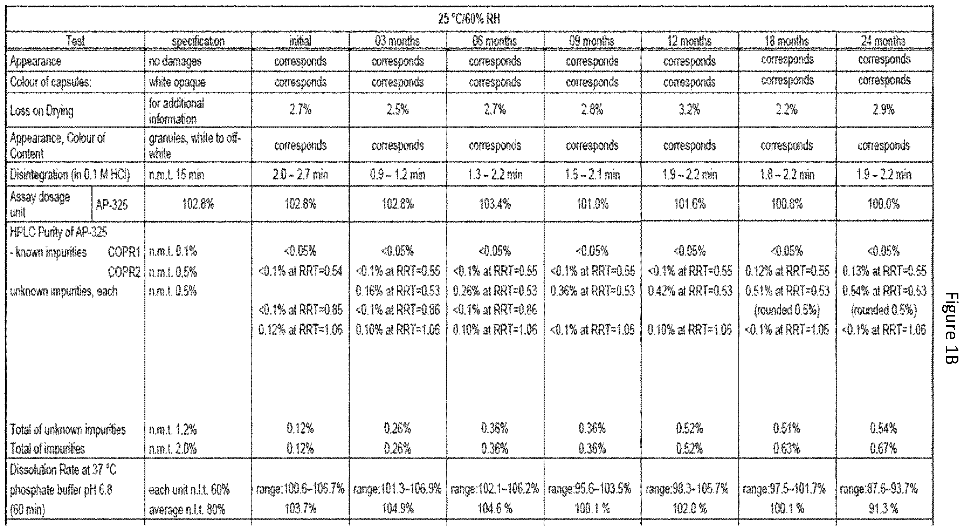

The stability parameters PSD and dissolution have been already described in [**063**], but further data are as well important to represent a good stability of a dosage form. These stability data are summarized in figure 1b and demonstrate that the AP-325 nanoparticulate composition is unaffected under the described ICH storage conditions to any changes of the investigated parameters and can be regarded as very stable over the investigated storage period.

### 7. Rat study protocol (pk study, see also figures 5a) and b)

### Application

p.o. (gavage) as single dose

### Animal Specification

| | |
|---|---|
| Species: | Sprague Dawley rats, fastened |
| Sex: | Males |

### Experimental groups and doses

Animals were treated with a single dose according to table 16a and 16b:

**Table 16a:**

| Experimental groups and doses (study 1) | | | | | |
|---|---|---|---|---|---|
| **No.** | **Group** | **No. and sex of animals** | **Route of administration** | **Dose [mg/kg]** | **Administration volume [ml/kg]** |
| 1 | N031 | 8 males | oral | 10 | 3 |
| 2 | N032 | 8 males | oral | 10 | 3 |
| 3 | N033 | 8 males | oral | 10 | 3 |
| 4 | N034 | 8 males | oral | 10 | 3 |

**Table 16b:**

| Experimental groups and doses (study 2) | | | | | |
|---|---|---|---|---|---|
| **No.** | **Group** | **No. and sex of animals** | **Route of administration** | **Dose [mg/kg]** | **Administration volume [ml/kg]** |
| 1 | N060 (N039) | 8 males | oral | 10 | 2 |
| 2 | N061 (N042) | 8 males | oral | 10 | 2 |
| 3 | N062 (N055) | 8 males | oral | 10 | 2 |
| 4 | N063 (N014) | 8 males | oral | 10 | 2 |
| 5 | N064 (N059) | 8 males | oral | 10 | 2 |

The total volume to be administered was calculated according to the individual body weight recorded on the day of administration. Blood sampling was performed at several time points within 24h.

Blood samples approx. 600 µl were withdrawn using a butterfly and capillaries from the *tail vein.* The collected blood was immediately transferred into lithium heparin-containing tubes (e.g. Saarstedt), shaken by hand and stored for 30 minutes on crushed ice until centrifugation (2.500 x g and 4°C for 10 minutes). The supernatant plasma was separated and transferred into pre-labeled plastic tubes. The plasma samples were stored in an ultra-freezer (-80°C) until shipment.

### Analysis

For the pharmacokinetic studies of AP-325 in rats, a specific HPLC-MS/MS method was developed and validated for the quantification of AP-325 in rat plasma.

### 8. Dog study protocol (see also figure 6)

### Application

p.o. as single dose

### Animal Specification

| | |
|---|---|
| Species: | Beagle dogs |
| Sex: | 4 Males each |

### Experimental groups and doses

| | | |
|---|---|---|
| Group: | 2 | |
| Dose: | | 100 mg/ animal |

Blood samples were withdrawn, and the collected blood was immediately transferred into lithium heparin-containing tubes, shaken by hand and stored for 30 minutes on crushed ice until centrifugation (2.500 x g and 4°C for 10 minutes). The supernatant plasma was separated and transferred into pre-labeled plastic tubes. The plasma samples were stored in an ultra-freezer (-80°C) until shipment.

### Analysis

For the pharmacokinetic studies of AP-325 in dogs, a specific HPLC-MS/MS method was developed and validated for the quantification of AP-325 in dog plasma.

### 9. Phase 1 study protocol humans (Figure 7)

| | |
|---|---|
| Application: | single dose of 5, 15, 40, 100 and 150 mg of AP-325 (nanoparticulate composition) |
| Design: | randomised, placebo controlled, double blind |
| Subjects: | Healthy white male subjects between 18-45 years; BMI with 18 to 29.9 kg/m²; body weight ≥ 70 kg |

### Analysis

For the pharmacokinetic studies of AP-325 in humans, a specific HPLC-MS/MS method was developed and validated for the quantification of AP-325 in human plasma.

The invention relates to a nanoparticulate of AP-325 or a AP-325 derivative composition comprising: (a) particles of AP-325 or its derivative having an effective average particle size of less than about 2000 nm; and (b) at least one surface and/or polymeric stabilizer.

Furthermore, the invention relates to a pharmaceutical composition comprising said composition in combination with at least one pharmaceutically acceptable excipient.

Furthermore, the invention relates to the use of said pharmaceutical composition for preparation of a medicament

Moreover, the invention relates to a method of making a nanoparticulate AP-325 or an AP-325 derivative composition comprising contacting particles of AP-325 or an AP-325 derivative with at least one surface and/or polymeric stabilizer for a time and under conditions sufficient to provide a composition comprising AP-325 or a AP-325 derivative particles having an effective average particle size of less than about 2 microns.

A further aspect of the invention is a system for the treatment of certain diseases in a subject comprising administering to a subject of an effective amount of a composition comprising (a) particles of AP-325 or derivatives thereof having an average effective particle size of less than about 2000 nm, and (b) at least one surface and/or polymeric stabilizer. The nanoparticulate composition in this system can have a reduced Tₘₐₓ, a higher Cₘₐₓ and higher AUC in mammalian subjects compared to a composition containing AP-325 or one of its derivatives having an average effective particle size of more than about 2000 nm. In this system the nanoparticulate composition does not have a disintegration time of less or equal to 3 minutes. The system may further comprise one or more active agents useful for the treatment of certain human diseases.

## Claims

1. A nanoparticulate composition comprising
(a) particles comprising at least one active ingredient, wherein the particles have an effective average particle size in the range from about 70 nm to about 220 nm; and
(b) at least one surface stabilizer and/or at least one polymeric stabilizer, the composition comprises
(aa) particles of at least one active ingredient selected from the group consisting of (Z)-2-cyano-3-cyclopropyl-3-hydroxy-N-(3-methyl-4-(trifluoromethyl)phenyl) prop-2-enamide, (Z)-2-cyano-3-hydroxy-*N*-[4-(trifluoromethyl)phenyl]hept-2-en-6-ynamide, 2-cyano-3-cyclopropyl-N-(4-fluorophenyl)-3-hydroxyacrylamide, and salts thereof, and
(bb) at least one surface stabilizer and/or at least one polymeric stabilizer, and wherein the effective average particle size is measured by laser light scattering, wherein the effective average particle size is meant that at least 50% of said active agent particles have a particle size, by weight (volume based), of less than the effective average particle size.

2. The composition of claim 1, wherein the effective average particle size is in the range from about 90 nm to about 210 nm, more preferably in the range from about 100 nm to about 200 nm,

3. The composition of claims 1 or 2, wherein the at least one active ingredient is present in an amount in the range from about 99.5% to about 0.001%, preferably in the range from about 95% to about 0.1%, and more preferably in the range from about 90% to about 0.5%, by weight, based on the total combined dry weight of the active ingredient and the at least one surface stabilizer and/or polymeric stabilizer, not including other excipients.

4. A pharmaceutical composition comprising a composition according to any one of claims 1 to 3 in combination with at least one pharmaceutically acceptable excipient.

5. A method of making a composition of one or several of claims 1 to 3 comprising contacting particles of the at least one active ingredient with at least one surface stabilizer and/or at least one polymeric stabilizer for a time and under conditions sufficient to provide a composition comprising particles of the active ingredient having an effective average particle size as defined in one of claims 1 or 2.

6. A method of making a solid oral dosage form in which the nanoparticles from the composition of claim 1 containing one or several of the active ingredients are bound on a suitable pharmaceutical excipient or carrier by using a fluid bed drying process, a spray drying process, an extrusion process or a granulation process.

7. A system for use in the treatment or the prevention of neuropathic pain and/or central nervous system trauma related disorder and/or certain other diseases in a subject comprising administering to a subject of an effective amount of the composition of one or several of claims 1 to 3 or the pharmaceutical composition of claim 4.

8. The system for use of claim 7, wherein the nanoparticulate composition has a reduced Tₘₐₓ, a higher cₘₐₓ and higher AUC in mammalian subjects compared to a composition containing the one or several active ingredients as claimed in claim 1 having an average effective particle size of more than about 2000 nm.

9. The system for use of one of claims 7 and 8, wherein the nanoparticulate composition does not have a disintegration time of less or equal to 3 min.

10. The system for use of one or several of claims 7 to 9, further comprising one or more additional active agents useful for the treatment of certain human diseases.

11. The system for use of one of claims 7 to 10, wherein the system is used for the treatment of wherein the compound is used in the treatment of peripheral and/or predominantly peripheral neuropathic pain or central and/or predominantly central neuropathic pain.

12. The system for use of any one of claims 7 to 10, wherein said system is used to treat an inflammatory disease, type I diabetes and/or type II diabetes or an inflammatory disease.

## Patentansprüche

1. Nanopartikuläre Zusammensetzung, umfassend
(a) Partikel, die mindestens einen Wirkstoff umfassen, wobei die Partikel eine effektive durchschnittliche Partikelgröße im Bereich von etwa 70 nm bis etwa 220 nm aufweisen; und
(b) mindestens einen Oberflächenstabilisator und/oder mindestens einen polymeren Stabilisator, wobei die Zusammensetzung Folgendes umfasst:
(aa) Partikel mindestens eines Wirkstoffs, der aus der Gruppe ausgewählt ist, die aus [Z]-2-Cyano-3-cyclopropyl-3-hydroxy-N-(3-methyl-4-(trifluormethyl)phenyl)prop-2-enamid, (Z)-2-Cyano-3-hydroxy-*N*-[4-(trifluormethyl)phenyl]hept-2-en-6-ynamid, 2-Cyano-3-cyclopropyl-N-(4-fluorphenyl)-3-hydroxyacrylamid und Salzen davon besteht, und
(bb) mindestens einen Oberflächenstabilisator und/oder mindestens einen polymeren Stabilisator, und wobei die effektive durchschnittliche Partikelgröße durch Laserlichtstreuung gemessen wird, wobei die effektive durchschnittliche Partikelgröße bedeutet, dass mindestens 50 % der Wirkstoffpartikel eine Partikelgröße, bezogen auf das Gewicht (auf Volumenbasis), von weniger als der effektiven durchschnittlichen Partikelgröße aufweisen.

2. Zusammensetzung nach Anspruch 1, wobei die effektive durchschnittliche Partikelgröße im Bereich von etwa 90 nm bis etwa 210 nm, bevorzugter im Bereich von etwa 100 nm bis etwa 200 nm liegt.

3. Zusammensetzung nach Anspruch 1 oder 2, wobei der mindestens eine Wirkstoff in einer Menge im Bereich von etwa 99,5 % bis etwa 0,001 %, vorzugsweise im Bereich von etwa 95 % bis etwa 0,1 % und bevorzugter im Bereich von etwa 90 % bis etwa 0,5 %, bezogen auf das kombinierte Gesamttrockengewicht des Wirkstoffs und des mindestens einen Oberflächenstabilisators und/oder polymeren Stabilisators, nicht einschließlich anderer Hilfsstoffe, vorhanden ist

4. Pharmazeutische Zusammensetzung, umfassend eine Zusammensetzung nach einem der Ansprüche 1 bis 3 in Kombination mit mindestens einem pharmazeutisch verträglichen Hilfsstoff.

5. Verfahren zur Herstellung einer Zusammensetzung nach einem oder mehreren der Ansprüche 1 bis 3, umfassend das Kontaktieren von Partikeln des mindestens einen Wirkstoffs mit mindestens einem Oberflächenstabilisator und/oder mindestens einem polymeren Stabilisator für eine Zeit und unter Bedingungen, die ausreichen, um eine Zusammensetzung bereitzustellen, die Partikel des Wirkstoffs mit einer effektiven durchschnittlichen Partikelgröße, wie in einem der Ansprüche 1 oder 2 definiert, umfasst

6. Verfahren zur Herstellung einer festen oralen Dosierungsform, bei dem die Nanopartikel aus der Zusammensetzung nach Anspruch 1, die einen oder mehrere der Wirkstoffe enthalten, an einen geeigneten pharmazeutischen Hilfsstoff oder Träger gebunden werden, indem ein Wirbelschichttrocknungsverfahren, ein Sprühtrocknungsverfahren, ein Extrusionsverfahren oder ein Granulationsverfahren verwendet wird.

7. System zur Verwendung bei der Behandlung oder Prävention von neuropathischen Schmerzen und/oder traumabedingten Störungen des zentralen Nervensystems und/oder bestimmten anderen Krankheiten bei einem Subjekt, umfassend die Verabreichung einer wirksamen Menge der Zusammensetzung nach einem oder mehreren der Ansprüche 1 bis 3 oder der pharmazeutischen Zusammensetzung nach Anspruch 4 an ein Subjekt.

8. System zur Verwendung nach Anspruch 7, wobei die nanopartikuläre Zusammensetzung eine reduzierte Tₘₐₓ, eine höhere Cₘₐₓ und eine höhere AUC bei Säugetieren im Vergleich zu einer Zusammensetzung aufweist, die den einen oder die mehreren Wirkstoffe nach Anspruch 1 mit einer durchschnittlichen effektiven Partikelgröße von mehr als etwa 2000 nm enthält.

9. System zur Verwendung nach einem der Ansprüche 7 und 8, wobei die nanopartikuläre Zusammensetzung keine Zerfallszeit von weniger oder gleich 3 min aufweist.

10. System zur Verwendung nach einem oder mehreren der Ansprüche 7 bis 9, ferner umfassend einen oder mehrere zusätzliche Wirkstoffe, die zur Behandlung bestimmter menschlicher Krankheiten nützlich sind.

11. System zur Verwendung nach einem der Ansprüche 7 bis 10, wobei das System zur Behandlung von verwendet wird, wobei die Verbindung bei der Behandlung von peripheren und/oder überwiegend peripheren neuropathischen Schmerzen oder zentralen und/oder überwiegend zentralen neuropathischen Schmerzen verwendet wird.

12. System zur Verwendung nach einem der Ansprüche 7 bis 10, wobei das System verwendet wird, um eine entzündliche Erkrankung, Typ-I-Diabetes und/oder Typ-II-Diabetes oder eine entzündliche Erkrankung zu behandeln.

## Revendications

1. Composition nanoparticulaire comprenant
(a) des particules comprenant au moins une substance active, dans laquelle les particules ont une taille moyenne effective de particules comprise entre environ 70 nm et environ 220 nm ; et
(b) au moins un stabilisant de surface et/ou au moins un stabilisant polymère, la composition comprend
(aa) des particules d'au moins une substance active choisie dans le groupe constitué par le [Z]-2-cyano-3-cyclopropyl-3-hydroxy-N-(3-méthyl-4-(trifluorométhyl)phényl) prop-2-énamide, le (*Z*)-2-cyano-3-hydroxy-*N*-[4-(trifluorométhyl) phényl]hept-2-én-6-ynamide, le 2-cyano-3-cyclopropyl-N-(4-fluorophényl)-3-hydroxyacrylamide, et leurs sels, et
(bb) au moins un stabilisant de surface et/ou au moins un stabilisant polymère, et dans laquelle la taille moyenne effective de particules est mesurée par diffusion de lumière laser, dans laquelle la taille moyenne effective de particules signifie qu'au moins 50 % desdites particules d'agent actif ont une taille de particule, en poids (basé sur le volume), inférieure à la taille moyenne effective de particules.

2. Composition selon la revendication 1, dans laquelle la taille moyenne effective de particules est comprise dans la plage comprise entre environ 90 nm et environ 210 nm, plus préférablement dans la plage comprise entre environ 100 nm et environ 200 nm.

3. Composition selon la revendication 1 ou 2, dans laquelle l'au moins une substance active est présente en une quantité dans la plage comprise entre environ 99,5 % et environ 0,001 %, de préférence dans la plage comprise entre environ 95 % et environ 0,1 %, et plus préférablement dans la plage comprise entre environ 90 % et environ 0,5 %, en poids, sur la base du poids sec total combiné de la substance active et de l'au moins un stabilisant de surface et/ou stabilisant polymère, sans inclure d'autres excipients.

4. Composition pharmaceutique comprenant une composition selon l'une quelconque des revendications 1 à 3, en combinaison avec au moins un excipient pharmaceutiquement acceptable.

5. Procédé de fabrication d'une composition selon l'une ou plusieurs des revendications 1 à 3, comprenant la mise en contact de particules de l'au moins une substance active avec au moins un stabilisant de surface et/ou au moins un stabilisant polymère pendant une durée et dans des conditions suffisantes pour fournir une composition comprenant des particules de la substance active ayant une taille moyenne efficace de particules telle que définie dans l'une des revendications 1 ou 2.

6. Procédé de fabrication d'une forme galénique orale solide dans lequel les nanoparticules de la composition selon la revendication 1 contenant une ou plusieurs des substances actives sont liées sur un excipient ou support pharmaceutique approprié en utilisant un processus de séchage à lit fluidisé, un processus de séchage par pulvérisation, un processus d'extrusion ou un processus de granulation.

7. Système destiné à être utilisé dans le traitement ou la prévention de la douleur neuropathique et/ou d'un trouble lié à un traumatisme du système nerveux central et/ou de certaines autres maladies chez un sujet, comprenant l'administration à un sujet d'une quantité efficace de la composition selon l'une ou plusieurs des revendications 1 à 3 ou de la composition pharmaceutique selon la revendication 4.

8. Système destiné à être utilisé selon la revendication 7, dans lequel la composition nanoparticulaire a une Tₘₐₓ réduite, une Cₘₐₓ plus élevée et une ASC plus élevée chez des sujets mammifères par rapport à une composition contenant l'une ou plusieurs substances actives selon la revendication 1 ayant une taille moyenne effective de particules supérieure à environ 2000 nm.

9. Système destiné à être utilisé selon l'une des revendications 7 et 8, dans lequel la composition nanoparticulaire n'a pas un temps de désintégration inférieur ou égal à 3 min.

10. Système destiné à être utilisé selon l'une ou plusieurs des revendications 7 à 9, comprenant en outre un ou plusieurs agents actifs supplémentaires utiles pour le traitement de certaines maladies humaines.

11. Système destiné à être utilisé selon l'une des revendications 7 à 10, dans lequel le système est utilisé pour le traitement de dans lequel le composé est utilisé dans le traitement de la douleur neuropathique périphérique et/ou principalement périphérique ou de la douleur neuropathique centrale et/ou principalement centrale.

12. Système destiné à être utilisé selon l'une quelconque des revendications 7 à 10, dans lequel ledit système est utilisé pour traiter une maladie inflammatoire, le diabète de type I et/ou le diabète de type II ou une maladie inflammatoire.
